# EUROPEAN PATENT APPLICATION

(11) **EP 4 438 598 A1**
(43) Date of publication of application: **02.10.2024**
(21) Application number: 22896171.0
(22) Date of filing: 22.11.2022
(51) Int. Cl.: C07D 295/033, A61K 31/495

(54) **METHOD FOR PREPARING ARYLETHENE COMPOUND**

(30) Priority: 22.11.2021 KR 20210161115
(71) Applicant: Novmetapharma Co., Ltd., Seoul 06050 (KR)
(72) Inventor: JUNG, Hoe Yune, Pohang-si, Gyeongsangbuk-do 37668 (KR); LEE, Heonjong, Incheon 22760 (KR)
(74) Representative: V.O.
(86) International application number: PCT/KR2022/018469
(87) International publication number: WO 2023/090974

(57) **Abstract**

Provided is a process for preparing an aryl ethene compound having high purity with high yield via a simple process.

## Description

### [Technical Field]

The present invention relates to a process for preparing an aryl ethene compound. More particularly, the present invention relates to a process for preparing an aryl ethene compound having high purity with high yield.

### [Background Art]

The estrogen related receptor (ERR) is one of orphan nuclear receptors, meaning the identity of its endogenous ligand has not yet been determined. There are three types thereof, ERRα, ERRβ, and ERRγ, each with a different activation position.

Korean Patent Publication No. 10-2018-0001240 discloses that the aryl ethene compound represented by the following formula (1) has very high inhibitory activity against ERRγ and it can be used to prevent or treat diseases mediated by ERRγ, especially metabolic diseases such as obesity, diabetes, hyperlipidemia, fatty liver, or atherosclerosis, and retinopathy. It also discloses that the expression of endogenous ERRγ protein can be controlled to regulate MAP kinase (mitogen-activated protein kinase), and the function of NIS (sodium iodide symporter) can be improved to increase membrane-localized NIS, thereby promoting radioactive iodine intake in the treatment of thyroid cancer.

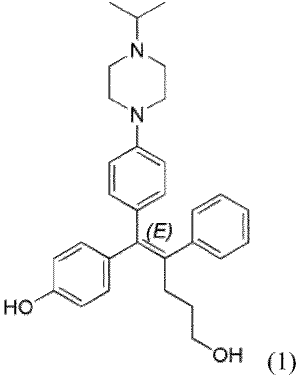

Therefore, there is a need for a method to obtain a large amount of the aryl ethene compound represented by the above formula (1). However, according to the conventional methods of preparing the aryl ethene compound, a large amount of Z isomer is produced along with the desired E isomer, and the process is complicated and uneconomical. Accordingly, there has been an urgent need to develop a method that can stereoselectively prepare the E isomer and increase the yield and economic efficiency through a simple process.

### [Disclosure]

### [Technical Problem]

It is an object of the present invention to provide a process for stereoselectively preparing an aryl ethene compound represented by formula (1).

It is another object of the present invention to provide a process for preparing an aryl ethene compound represented by formula (1) with high purity and high yield through a simple process.

### [Technical Solution]

One embodiment of the present invention relates to a process for preparing a compound of the following formula (1), which comprises the steps of:
(i) subjecting a compound of the following formula (2) to Friedel-Crafts reaction with a compound of the following formula (3) to obtain a compound of the following formula (4);
(ii) subjecting the compound of the following formula (4) to coupling reaction with a compound of the following formula (5) to obtain a compound of the following formula (6);
(iii) subjecting the compound of the following formula (6) to demethylation to obtain a compound of the following formula (7);
(iv) reacting the compound of the following formula (7) with pivaloyl chloride to obtain a compound of the following formula (8);
(v) subjecting the compound of the following formula (8) to McMurry reaction with a compound of the following formula (9) to obtain a compound of the following formula (10); and
(vi) subjecting the compound of the following formula (10) to reduction.

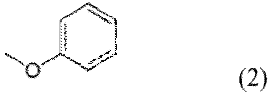

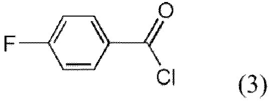

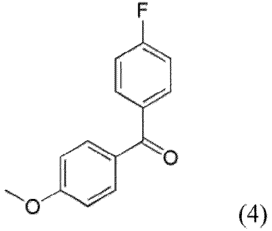

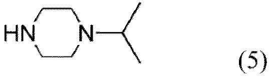

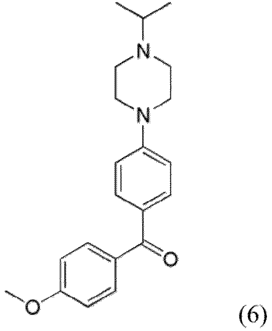

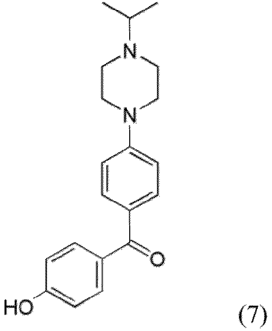

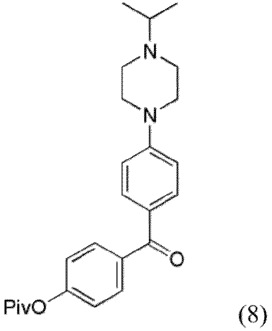

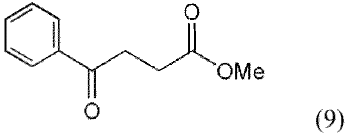

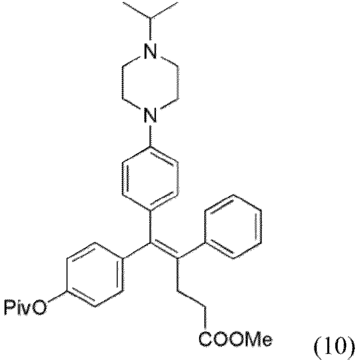

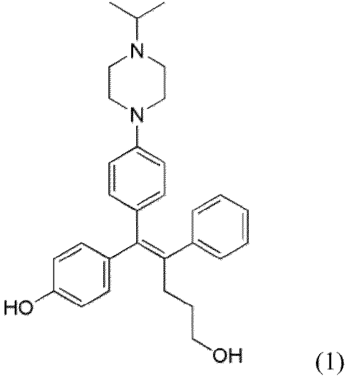

Hereinafter, the preparation process of the present invention is described in more detail referring to the following reaction scheme 1. The process depicted in the following reaction scheme 1 represents merely a typical example, and the reagents, reaction conditions, etc. may be changed depending on the case without limitation.

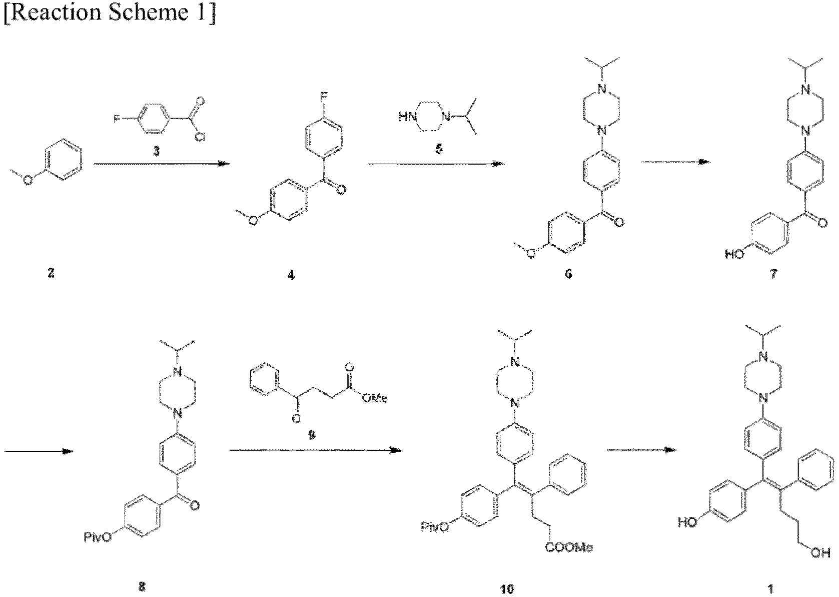

### Step 1: Synthesis of Compound of Formula (4)

The compound of formula (4) can be prepared by subjecting the compound of formula (2) to Friedel-Crafts reaction with the compound of formula (3).

The Friedel-Crafts reaction may be carried out in the presence of a Lewis acid.

As the Lewis acid, aluminum chloride, aluminum bromide, or the like may be used, and aluminum chloride (AlCl₃) is particularly preferred.

The reaction temperature is preferably room temperature, and dichloromethane, diethyl ether, or the like can be used as the reaction solvent.

### Step 2: Synthesis of Compound of Formula (6)

The compound of formula (6) can be prepared by subjecting the compound of formula (4) to coupling reaction with the compound of formula (5) in the presence of a base.

As the base, potassium carbonate, cesium carbonate, sodium carbonate, or the like may be used, with potassium carbonate (K₂CO₃) being particularly preferred.

The base may be used in an amount of 1.5 to 2.5 equivalents, preferably about 2 equivalents, to 1 equivalent amount of the compound of formula (4).

The reaction temperature is preferably 140 to 180 °C, more preferably 150 to 170 °C.

As the reaction solvent, N-methylpyrrolidone, dimethyl sulfoxide, or the like may be used, and N-methylpyrrolidone (NMP) is particularly preferred.

### Step 3: Synthesis of Compound of Formula (7)

The compound of formula (7) can be prepared by subjecting the compound of formula (6) to demethylation.

The demethylation can be carried out using HBr, HCl, boron tribromide, aluminum tribromide, or the like, and HBr is particularly preferred.

At this time, the reaction temperature is preferably under reflux conditions, and acetic acid (AcOH) or the like may be used as the reaction solvent.

### Step 4: Synthesis of Compound of Formula (8)

The compound of formula (8) can be prepared by reacting the compound of formula (7) with pivaloyl chloride (PivCl) in the presence of a base.

As the base, triethylamine, 4-dimethylaminopyridine, imidazole, 2,6-rutidine, or the like can be used, and triethylamine (TEA) is particularly suitable.

At this time, the reaction temperature is preferably room temperature, and dichloromethane, chloroform, or the like may be used as the reaction solvent.

### Step 5: Synthesis of Compound of Formula (10)

The compound of formula (10) can be prepared by subjecting the compound of formula (8) to McMurry reaction with the compound of formula (9).

The McMurry reaction may be carried out in the presence of TiCl₄ and Zn.

At this time, the reaction temperature is preferably under reflux conditions, and the reaction solvent may be tetrahydrofuran, dichloromethane, toluene, or the like, and tetrahydrofuran is particularly preferred.

According to one embodiment of the present invention, the stereoselectivity can be increased by introducing bulky substituents and performing McMurry reaction to suppress the generation of the Z isomer. Thus, only the E isomer can be prepared with high purity in the subsequent step of preparing a hydrochloride salt described below.

### Step 6: Synthesis of Compound of Formula (1)

The compound of formula (1) can be prepared by subjecting the compound of formula (10) to reduction.

The reduction can be carried out using lithium aluminum tetrahydride, sodium bis(2-methoxyethoxy)aluminum dihydride, diisobutylaluminum hydride (DIBAL-H), or the like. In particular, it is preferably carried out using lithium aluminum tetrahydride (LAH).

At this time, the reaction temperature is preferably about 0°C. As the reaction solvent, tetrahydrofuran, methylene chloride, toluene, or the like can be used, and tetrahydrofuran is particularly preferred.

One embodiment of the present invention relates to a process for preparing a dihydrochloride salt of the compound of formula (1) by reacting the compound of formula (1) obtained by the above preparation process with hydrogen chloride.

In this case, the reaction temperature is preferably room temperature, and methanol, 1,4-dioxane, or the like may be used as the reaction solvent, and methanol is particularly preferred.

The dihydrochloride salt of the compound of formula (1) prepared in this manner has a purity of at least 99.3%, indicating that the Z isomer has been effectively removed.

### [Advantageous Effects]

In accordance with the preparation process of the present invention, the aryl ethene compound represented by formula (1) having high purity can be mass-produced with high yields through a simple process.

### [Best Mode]

The present invention will be described in more detail by the following examples. It will be obvious to those skilled in the art that these examples are merely described for illustration of the present invention and the scope of the present invention is not limited thereto.

### Example 1: Preparation of Compound of Formula (4)

The compound of formula (2) (12.5 kg, 115 mol, 1.00 *eq*) and the compound of formula (3) (18.4 kg, 115 mol, 1.00 *eq*) were dissolved in dichloromethane (165 kg), and AlCl₃ (16.2 kg, 121 mol, 1.05 *eq*) was added at -5 to 5 °C. The reaction mixture was stirred at 20 to 25 °C for 10 hours, then added to HCl solution (aq, 1.60 mol·L⁻¹, 100 L, 160 mol), and after layer separation, the aqueous layer was extracted with dichloromethane (50 L). The organic layers were combined and washed with NaHCO₃ (50 L), followed by saline (50 L). Then the resulting solution was dried over anhydrous Na₂SO₄, filtered, and concentrated under reduced pressure. The residue was treated with petroleum ether (50 L), filtered, and concentrated to obtain the compound of formula (4) (22.9 kg, 99.5 mol, 86.0% yield) as a red solid.

¹H NMR (400 MHz, CDCl₃): 7.80 (td, J = 2.6, 8.9 Hz, 4H), 7.20 - 7.12 (m, 2H), 7.02 - 6.94 (m, 2H), 3.90 (s, 3H)

### Example 2: Preparation of Compound of Formula (6)

The compound of formula (4) (6.15 kg, 26.7 mol, 1.00 *eq*) was dissolved in NMP (24.6 L, 4 vol), and the compound of formula (5) (5.91 kg, 29.4 mol, 1.10 *eq,* 2HCl) and K₂CO₃ (7.38 kg, 53.4 mol, 2.00 *eq*) were added. The reaction mixture was stirred at 160 °C for 24 hours, and then cooled to 25 °C. The reaction mixture was filtered, and the filter cake was washed with NMP (5.00 L). The organic layer was treated with H₂O (100 L) at 0 °C for 30 minutes, the reaction mixture was filtered, and the filter cake was washed with H₂O (25 L). The filter cake was hot air dried at 50 °C for 1 day to obtain the compound of formula (6) (8.00 kg, 26.7 mol, 88.4% yield) as a yellow solid.

¹H NMR (400 MHz, CDCl₃): 7.85 - 7.71 (m, 4H), 7.02 - 6.85 (m, 4H), 3.88 (s, 3H), 3.45 - 3.32 (m, 4H), 2.79 - 2.63 (m, 5H), 1.10 (d, J = 6.5 Hz, 6H)

### Example 3: Preparation of Compound of Formula (7)

The compound of formula (6) (32.0 kg, 94.5 mol, 1.00 *eq*) was dissolved in AcOH (80.0 L), and HBr (240 kg, 160 L, 48.0% purity) was added dropwise at 25 to 40 °C for 30 minutes. The reaction mixture was heated under reflux (110 °C) for 24 hours and quenched at 0 °C with water (300 L). The reaction mixture was filtered, and the filter cake was treated with EtOH (150 L) at 25 °C for 30 minutes. The reaction mixture was then filtered, and the filter cake was dried under reduced pressure to obtain the compound of formula (7) (37.0 kg, 91.7 mol, 96.6% yield) as a red solid.

¹H NMR (400 MHz, DMSO-d₆): 10.71 - 9.96 (m, 1H), 7.72 - 7.52 (m, 4H), 7.20 - 7.04 (m, 2H), 6.95 - 6.83 (m, 2H), 4.20 - 4.02 (m, 2H), 3.66 - 3.49 (m, 3H), 3.36 - 3.07 (m, 4H), 1.42 - 1.23 (m, 6H)

### Example 4: Preparation of Compound of Formula (8)

The compound of formula (7) (37.0 kg, 1.00 *eq*) and TEA (46.0 kg, 5.00 *eq*) were dissolved in dichloromethane (185 L), and PivCl (22.0 kg, 2.00 *eq*) was added dropwise at 0 °C for 30 minutes. The reaction mixture was stirred at 25 °C for 12 hours and quenched at 15 °C with water (100 L). Then it was extracted with CH₂Cl₂ (50 L*2), the organic layers were combined, washed with saline (50 L*2), dried over Na₂SO₄, filtered, and concentrated under reduced pressure. The residue was treated with petroleum ether (150 L) at 25 °C for 30 minutes. The reaction mixture was filtered, and the filter cake was dried under reduced pressure to obtain the compound of formula (8) (30.0 kg, 80.4% yield, 93.8% purity) as a white solid.

¹H NMR (400 MHz, CDCl₃): 7.84 - 7.74 (m, 4H), 7.22 - 7.11 (m, 2H), 6.95 - 6.85 (m, 2H), 3.49 - 3.32 (m, 4H), 2.84 - 2.62 (m, 5H), 1.42 - 1.36 (m, 9H), 1.15 - 1.07 (m, 6H)

### Example 5: Preparation of Compound of Formula (10)

Zn (9.60 kg, 3.00 *eq*) was dissolved in THF (100 L), TiCl₄ (1 M, 73.4 L, 1.47 mol, 1.50 *eq*) was added at 0 °C for 1 hour, and then the reaction mixture was stirred at 60 °C under reflux for 2 hours. A solution of the compound of formula (8) (20.0 kg, 1.00 *eq*) and the compound of formula (9) (11.3 kg, 1.20 *eq*) dissolved in THF (200 L) was added at 50 °C for 1 hour, and the reaction mixture was stirred at 50 °C under reflux for 3 hours. The reaction mixture was filtered, and the filtrate was added to 4 M HCl (120 L). The organic layer was washed with NH₃-H₂O (1 M) (100 L) and saline (100 L), dried over Na₂SO₄, filtered, and concentrated under reduced pressure. The residue was purified by silica gel column chromatography (dichloromethane/methanol = 1/0 to 200/1) to obtain the compound of formula (10) (8.4 kg, 30% yield) as a yellow solid.

¹H NMR (400 MHz, CDCl₃): 7.33 - 7.02 (m, 9H), 6.73 (d, J = 8.9 Hz, 2H), 6.57 (d, J = 8.8 Hz, 2H), 4.57 - 4.51 (m, 1H), 3.56 (s, 3H), 3.20 - 3.08 (m, 4H), 2.84 - 2.75 (m, 2H), 2.74 - 2.57 (m, 5H), 2.36 - 2.26 (m, 2H), 1.39 (s, 9H), 1.08 (d, J = 6.6 Hz, 6H)

### Example 6: Preparation of Compound of Formula (1)

LAH (267 g, 7.03 mol, 2.0 *eq*) was dissolved in THF (7.50 L) at 0 °C, then a solution of the compound of formula (10) (2.00 kg, 3.52 mol, 1.00 *eq*) dissolved in THF (7.50 L) was added dropwise at 0 °C for 0.5 hour. The reaction mixture was stirred at 0 °C for 0.5 hour, and a solution of seignette salt (1.00 kg) dissolved in 5.00 L of water was added at 0 °C for 2 hours and stirred at 25 °C for 1 hour. Water (15 L) was added to the reaction mixture and extracted with 30 L of CH₂Cl₂ (10 L * 3). The organic layers were combined, washed with 10 L of H₂O (5.0 L*2) and 5 L of saline, dried over anhydrous Na₂SO₄, filtered, and concentrated under reduced pressure. The residue was purified by silica gel column chromatography (dichloromethane/methanol = 20/1) to obtain the compound of formula (1) (3,000 g, 6.44 mol, 61.03% yield, 98% purity) as a white solid.

¹H NMR (400 MHz, MeOD-d₄): 7.17 - 6.99 (m, 7H), 6.79 - 6.70 (m, 4H), 6.64 - 6.57 (m, 2H), 3.40 (t, J = 6.8 Hz, 2H), 3.12 - 3.03 (m, 4H), 2.73 - 2.62 (m, 5H), 2.56 - 2.45 (m, 2H), 1.60 - 1.48 (m, 2H), 1.14 - 1.06 (m, 6H), 0.97 - 0.91 (m, 1H)

### Example 7: Preparation of Dihydrochloride Salt of Compound of Formula (1)

The compound of formula (1) (3.00 kg, 6.57 mol, 1.00 *eq*) was dissolved in MeOH (30.0 L), and HCl/MeOH (15 M, 1.75 L, 4.00 *eq*) was added at 0 °C for 10 minutes. The reaction mixture was stirred at 25 °C for 20 minutes, filtered, and the filter cake was dried under reduced pressure to obtain the dihydrochloride salt of the compound of formula (1) (3.29 kg, 6.17 mol, 93.9% yield, 99.3% purity, 2HCl) as a white solid.

¹H NMR (400 MHz, MeOD-d₄): 7.19 - 6.99 (m, 7H), 6.91 - 6.81 (m, 4H), 6.81 - 6.74 (m, 2H), 3.78 (br d, J = 13.1 Hz, 2H), 3.64 - 3.53 (m, 3H), 3.45 - 3.32 (m, 5H), 3.29 - 3.19 (m, 2H), 2.57 - 2.47 (m, 2H), 1.60 - 1.49 (m, 2H), 1.40 (d, J = 6.7 Hz, 6H)

## Claims

1. A process for preparing a compound of the following formula (1), which comprises the steps of:
(i) subjecting a compound of the following formula (2) to Friedel-Crafts reaction with a compound of the following formula (3) to obtain a compound of the following formula (4);
(ii) subjecting the compound of the following formula (4) to coupling reaction with a compound of the following formula (5) to obtain a compound of the following formula (6);
(iii) subjecting the compound of the following formula (6) to demethylation to obtain a compound of the following formula (7);
(iv) reacting the compound of the following formula (7) with pivaloyl chloride to obtain a compound of the following formula (8);
(v) subjecting the compound of the following formula (8) to McMurry reaction with a compound of the following formula (9) to obtain a compound of the following formula (10); and
(vi) subjecting the compound of the following formula (10) to reduction:

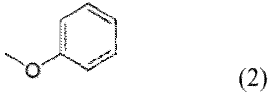

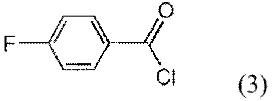

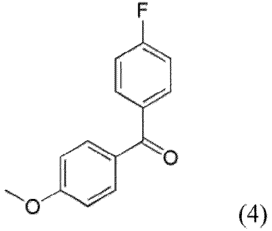

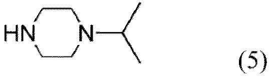

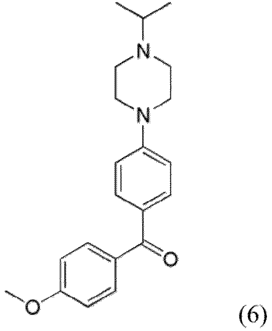

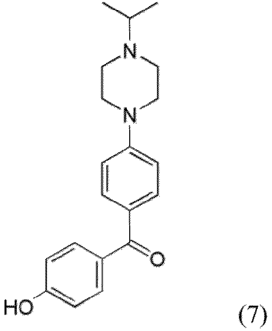

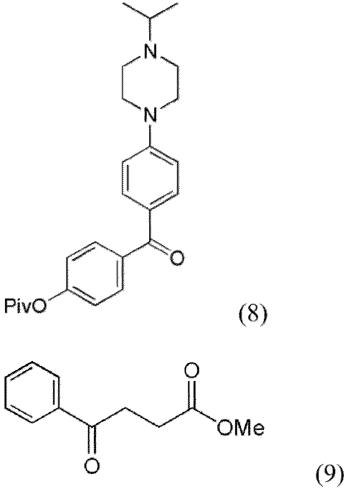

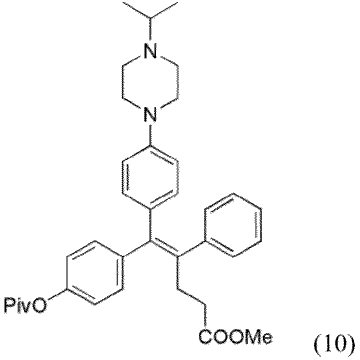

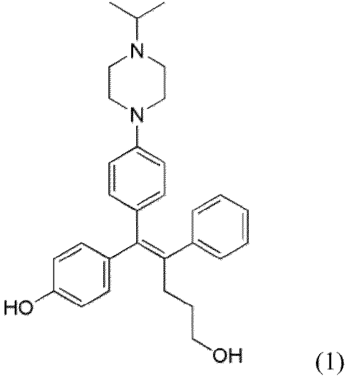

2. The process according to claim 1, wherein the Friedel-Crafts reaction of step (i) is carried out in the presence of aluminum chloride.

3. The process according to claim 1, wherein the coupling reaction of step (ii) is carried out in the presence of potassium carbonate in N-methylpyrrolidone.

4. The process according to claim 1, wherein the demethylation of step (iii) is carried out using HBr and acetic acid.

5. The process according to claim 1, wherein the McMurry reaction of step (v) is carried out in the presence of TiCl₄ and Zn.

6. The process according to claim 1, wherein the reduction of step (vi) is carried out using lithium aluminum tetrahydride.

7. A process for preparing a dihydrochloride salt of the compound of formula (1), comprising the step of reacting the compound of formula (1) prepared by the process according to any one of claims 1 to 6 with hydrogen chloride.
